# EUROPÄISCHE PATENTSCHRIFT

(11) **EP 3 515 248 B1**
(45) Veröffentlichungstag und Bekanntmachung des Hinweises auf die Patenterteilung: **02.09.2020**
(21) Anmeldenummer: 16770521.9
(22) Anmeldetag: 26.09.2016
(51) Int. Cl.: A47B 9/00

(54) **STEUERUNG EINES HÖHENVERSTELLBAREN TISCHES MITTELS FINGERABDRUCK**
FINGERPRINT CONTROL OF A HEIGHT-ADJUSTABLE TABLE
COMMANDE D'UNE TABLE RÉGLABLE EN HAUTEUR PAR UNE EMPREINTE DIGITALE

(43) Veröffentlichungstag der Anmeldung: 31.07.2019
(73) Patentinhaber: Kesseböhmer Produktions GmbH & Co. KG, 73235 Weilheim/Teck (DE)
(72) Erfinder: HANSEN, Melf, 75053 Gondelsheim (DE)
(74) Vertreter: Prüfer & Partner mbB Patentanwälte · Rechtsanwälte
(86) Internationale Anmeldenummer: PCT/EP2016/072858
(87) Internationale Veröffentlichungsnummer: WO 2018/054504

(56) Entgegenhaltungen:
- WO-A1-2010/073243
- WO-A1-2014/113873
- WO-A2-2005/017839
- US-A1- 2009 140 982

## Beschreibung

Aus dem Stand der Technik sind elektrisch höhenverstellbare Tische bekannt, die eine Eingabevorrichtung zum Bedienen einer Höhenverstellfunktion einer Tischplatte aufweisen, durch teleskopartiges Aus- bzw. Einfahren der Tischbeine. Wenn ein Anwender die Höhe der Tischplatte verstellen möchte, muss er einen dafür vorgesehenen Knopf oder Schalter an der Eingabevorrichtung, oder auch einen Button auf einem Display der Eingabevorrichtung, drücken und gedrückt zu halten. Die Tischplatte ändert daraufhin so lange ihre Höhe in die gewählte Richtung, bis sie entweder einen Endanschlag erreicht, oder bis der Anwender den Knopf oder Schalter loslässt.

An der Eingabevorrichtung ist in der Regel ein Knopf zum Hochfahren des Tisches vorgesehen, der mit einem Pfeil nach oben beschriftet ist, sowie ein Knopf zum Runterfahren des Tisches, mit einem Pfeil nach unten.

Weiterhin ist es bekannt, eine bevorzugte Sitz- und/oder Stehhöhe eines Anwenders in einem Steuergerät des höhenverstellbaren Tisches abzuspeichern und mittels einer Memoryfunktion bei Bedarf abzurufen. Hierfür weist das Eingabeelement mehrere Knöpfe auf, wobei durch Drücken eines Knopfes die jeweils hinterlegte Tischhöhe angefahren werden kann. Beispielsweise weist das Eingabeelement des Tisches einen Knopf mit der Beschriftung "M1" auf, sowie einen Knopf mit der Beschriftung "M2". Wenn nun ein Anwender mittels der Knöpfe "Hoch/Runter" seine bevorzugte Tischhöhe zum Sitzen eingestellt hat, kann er beispielsweise genau diese Höhe im Steuergerät abspeichern und dem Knopf "M1" zuordnen (bzw. hinterlegen). Falls daraufhin die Tischhöhe verstellt wird, kann der Benutzer durch Gedrückthalten des Knopfes "M1" bei Bedarf jederzeit wieder die gespeicherte bevorzugte Tischhöhe zum Sitzen anfahren. Hierbei ist es irrelevant, ob die aktuelle Tischhöhe unter oder über dem gespeicherten Wert (M1) liegt. Folglich kann durch Drücken und Gedrückthalten des beispielhaften Kopfes "M1" der Tisch entweder nach oben oder nach unten verfahren werden, in Abhängigkeit davon, ob die aktuelle Tischhöhe unter oder über der abgespeicherten Tischhöhe liegt.

Des Weiteren ist aus der internationalen Patentanmeldung WO2014/113873A1 eine Workstation einschließlich einer Tischplatte, eines Rahmens, einer an die Tischplatte und den Rahmen gekoppelten Stütze und eines motorisch angetriebenen Rotators bekannt, wobei der Rotator die Stütze und die Tischplatte horizontal zu der Position des Benutzers bewegt.

Außerdem ist aus der internationalen Patentanmeldung WO2005/017839A2 eine elektronische Vorrichtung mit mindestens einer Bedieneinheit zum Steuern der elektronischen Vorrichtung bekannt, wobei der Benutzer während des Betriebs der Bedieneinheit automatisch identifiziert wird und diesem je nach Identität des Benutzers Funktionen und/oder Funktionsauswahlen angeboten oder von diesem ausgeführt werden können.

Auf Grund von gesetzlichen Regularien dürfen höhenverstellbare Tische in Europa wegen fehlender redundanter Steuerungssysteme heutzutage nicht automatisch verstellt werden. Aus diesem Grund muss beispielsweise der obige Knopf "M1" während des gesamten Verstellvorgangs gedrückt und gehalten werden, obwohl das Steuergerät erkennen kann, in welche Endstellung der Tisch verstellt werden soll, nämlich auf die gespeicherte Höhe.

Es ergeben sich aus dem Stand der Technik dahingehend Probleme, wenn beispielsweise in einem Großraumbüro eine beliebige Anzahl von Mitarbeitern keinen festgelegten Arbeitsplatz (Schreibtisch) verwenden, wegen externer Projektarbeiten, Reisetätigkeiten, Homeoffice etc. Folglich wird ein Schreibtisch unter Umständen jeden Tag von einem anderen Mitarbeiter verwendet. Um die oben beschriebene Memoryfunktion auch in dieser Konstellation verwenden zu können, müsste jeder Schreibtisch für jeden Mitarbeiter einen entsprechenden Knopf aufweisen, auf dem die jeweils bevorzugte Tischhöhe gespeichert ist und abgerufen werden kann. Dies ist bei einer Vielzahl von Mitarbeitern allerdings nicht umsetzbar.

Um dieses Problem zu lösen, sind im Stand der Technik App-Lösungen zur Verwendung mittels eines Smartphones bekannt. Der Mitarbeiter kann demnach eine entsprechende App auf sein Smartphone laden und sich bei dem Steuergerät eines ausgewählten Schreibtischs anmelden, um diesen auf die individuell abgespeicherte Wunschhöhe einzustellen. Nachteilig hierbei ist, dass der Mitarbeiter vor der Verwendung des Tisches, falls dieser zum Hinsetzen zu niedrig eingestellt ist, erst die App aufrufen muss, und erst dann den Tisch über die App oder beispielsweise auch über einen Knopf am Schreibtisch verstellen kann, bevor der Mitarbeiter am dann korrekt eingestellten Tisch Platz nehmen kann.

Es ist eine Aufgabe der Erfindung, ein Verfahren, eine Vorrichtung, sowie ein System anzubieten, mit dem/der ein höhenverstellbarer Tisch einfach und komfortabel auf eine individuell für einen Benutzer abgespeicherte Wunschhöhe einstellbar ist.

Diese Aufgabe wird durch eine Ausgestaltung gemäß der unabhängigen Ansprüche gelöst. Vorteilhafte Weiterentwicklungen sind Gegenstand der Unteransprüche.

Durch das Ausstatten eines Bedienelements eines höhenverstellbaren Tischs mit einem Fingerabdrucksensor ist es möglich, einen Benutzer beim Bedienen beispielsweise einer Höhenverstellfunktion zu identifizieren. Des Weiteren ist es möglich, daraufhin einen Parameter für diesen Benutzer mittels eines Steuergeräts abzurufen, und die von dem Benutzer bediente Funktion personalisiert auszuführen. Beispielsweise kann eine vorher festgelegte bevorzugte Tischhöhe für diesen Benutzer angefahren werden.

Falls beispielsweise in einem Großraumbüro 20 Schreibtische für 20 Mitarbeiter angeordnet sind, aber kein Schreibtisch einem Mitarbeiter fest zugeordnet ist, kann gemäß des Standes der Technik kein Mitarbeiter die möglicherweise vorgesehene Memoryfunktion seines aktuellen Schreibtischs sinnvoll nutzen. Der Benutzer kann zwar an seinem aktuellen Schreibtisch eine bevorzugte Höhe der Tischplatte zum Sitzen einstellen und abspeichern, sowie eine bevorzugte Höhe der Tischplatte zum Stehen. Falls der Mitarbeiter allerdings am nächsten Tag an einem anderen der 20 Schreibtische seine Arbeit verrichtet, kann er die eingestellten bevorzugten Höhen der Tischplatte nicht mehr nutzen. Durch die erfindungsgemäße Ausgestaltung ist es möglich, verschiedene bevorzugte Tischhöhen für verschiedene Benutzer in einem Steuergerät eines Schreibtischs zu speichern. Wenn nun ein Benutzer an einem beliebigen Schreibtisch Platz nimmt, und mit seinem Finger den höhenverstellbaren Schreibtisch bedient, erkennt das Steuergerät des Schreibtischs erfindungsgemäß mittels eines Fingerabdrucksensors, welcher Benutzer die Bedienung ausführt, um daraufhin beispielsweise gespeicherte bevorzugte Tischplattenhöhen abrufen, und individuell für diesen Benutzer einzustellen.

Durch das vorzugsweise Vorsehen eines zentralen Servers, auf den eine beliebige Anzahl von Schreibtischen bzw. Steuergeräten zugreift, kann ein Datensatz an Parametern für verschiedene Benutzer zentral verwaltet und bei Bedarf zentral geändert werden. Somit ist es nicht nötig, die in jedem Steuergerät jedes Schreibtischs gespeicherten Datensätze separat zu verwalten bzw. zu aktualisieren.

Durch das Vorsehen eines vertikal angeordneten Kippschalters zum Bedienen ist eine intuitive Bedienung der Funktionen "hoch/runter" möglich. Des Weiteren muss nur ein einziger Fingerabdrucksensor unter diesem Kippschalter vorgesehen werden, um zwei verschiedene Funktionen personalisiert bedienen zu können.

Durch das Vorsehen von wenigstens zwei Erkennungsflächen muss zwar unter jeder Erkennungsfläche ein separater Fingerabdrucksensor vorgesehen werden, allerdings kann auch ein Bedienen von mehreren Erkennungsflächen gleichzeitig erkannt werden, anders als beim Kippschalter, der entweder nach oben oder nach unten gekippt werden kann.

Durch das vorzugsweise Kombinieren von Kippschalter bzw. Erkennungsfläche mit einem Fingerabdrucksensor kann ein Benutzer durch einen Bedienungsschritt eine Personalisierung sowie eine Funktion (z.B. Verstellfunktion) ausführen. Vorzugsweise weisen Kippschalter bzw. Erkennungsfläche eine transparente Fläche auf, unter der ein Fingerabdrucksensor angeordnet ist.

Durch das vorzugsweise Starten einer Verstellbewegung eines höhenverstellbaren Tischs noch bevor der bedienende Benutzer identifiziert wurde, und/und oder noch bevor die gespeicherten Parameter für den erkannten Benutzer ermittelt bzw. abgerufen wurden, ist ein besonders schnelles Ansprechverhalten beim Bedienen des höhenverstellbaren Tischs zu erreichen. Die unter Umständen entstehende Verzögerung auf Grund der Zeitspanne, bis der Benutzer identifiziert wurde und/und oder bis die gespeicherten Parameter ermittelt bzw. abgerufen wurden, werden von dem Benutzer hierbei nicht wahrgenommen, da der Verstellvorgang bereits unmittelbar nach Benutzereingabe erfolgt.

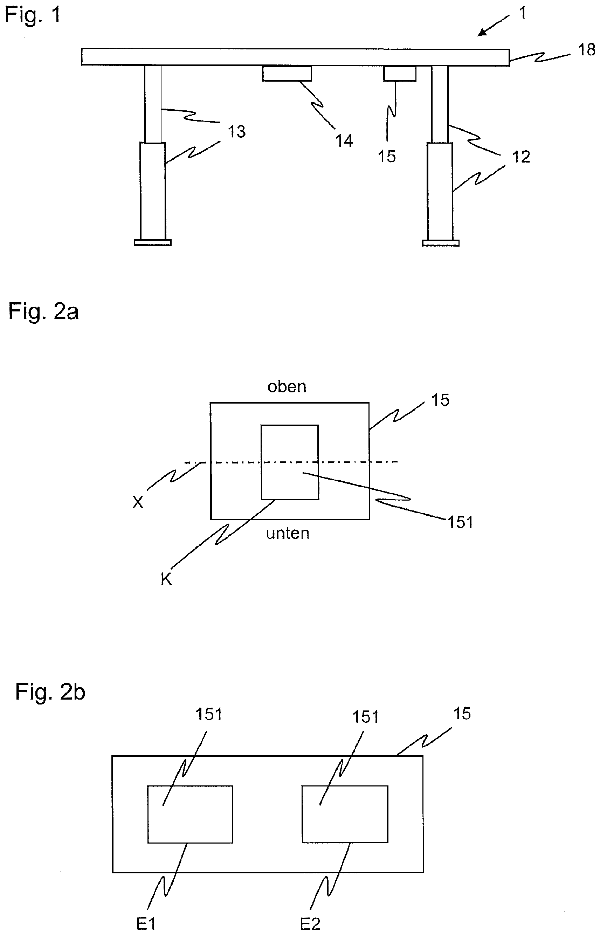

Figur 1 zeigt eine Ausführungsform eines höhenverstellbaren Tischs 1 mit einer Tischplatte 18 und zwei elektrisch höhenverstellbaren Säulen 12, 13. An der Unterseite der Tischplatte sind ein Steuergerät 14 und ein Bedienelement 15 angebracht. Auf mögliche Ausgestaltungen des Bedienelements 15 wird in Bezug auf die nachfolgenden Figuren eingegangen. In dieser Ausführungsform weist das Steuergerät 14 eine Speichervorrichtung 141 auf, die zum Abspeichern und Bereitstellen von Parametern eingerichtet ist. Das Steuergerät 14 ist dazu eingerichtet, die Parameter von der Speichervorrichtung 141 abzurufen (interne Kommunikation) und gegebenenfalls weiter zu verarbeiten. Des Weiteren weist das Steuergerät 14 eine Datenschnittstelle auf, zum Schreiben und Auslesen der Parameter auf bzw. von der Speichervorrichtung 141 von außerhalb (externe Kommunikation). Diese Datenschnittstelle entspricht in vorliegender Ausführungsform dem Typ USB.

In einer weiteren, nicht dargestellten Ausführungsform weist das Steuergerät 14 eine Datenschnittstelle auf, die zur (externen) Datenkommunikation mittels Funkverbindung (W-Lan und/oder Bluetooth) eingerichtet ist. Bei dieser Ausführungsform erfolgt ein externer Zugriff auf die Speichervorrichtung 141, zum Schreiben und Auslesen der Parameter, über eine Funkverbindung. Die restliche Konfiguration entspricht der Figurenbeschreibung zu Fig. 1.

In einer weiteren, nicht dargestellten Ausführungsform weist das Steuergerät 14 keine Speichervorrichtung 141, allerdings trotzdem eine Datenschnittstelle auf, zur (externen) Datenkommunikation. Die Datenschnittstelle entspricht dabei entweder einer kabelgebundenen Schnittstelle (beispielsweise USB), oder einer kabellosen Funkschnittstelle (W-Lan und/oder Bluetooth). Bei dieser Ausführungsform ist das Steuergerät 14 dazu eingerichtet, die Parameter genau dann mittels der Datenschnittstelle (extern) abzurufen, beispielsweise von einem Server (S), wenn diese von dem Steuergerät 14 benötigt werden. In dieser Ausführungsform kann das Steuergerät 14, obwohl es keine Speichervorrichtung 141 aufweist, einen Arbeitsspeicher aufweisen, zum temporären Zwischenspeichern der vom Server (S) abgerufenen Parameter. Die restliche Konfiguration entspricht der Figurenbeschreibung zu Fig. 1.

Bei den bisher beschriebenen Ausführungsformen weisen die Parameter personalisierte Informationen für eine beliebige Anzahl von Benutzer auf. Ein Parameter-Datensatz enthält wenigstens einen Fingerabdruck eines Benutzers, sowie eine beliebige Anzahl von weiteren Informationen zu genau diesem Benutzer. Diese weiteren Informationen umfassen beispielsweise eine bevorzugte Tischhöhe zum Sitzen an dem Tisch 1 (minimale Tischhöhe), sowie eine bevorzugte Tischhöhe zum Stehen an dem Tisch 1 (maximale Tischhöhe). Weitere Informationen für jeweils einen Benutzer können darüber hinaus unter anderem umfassen: eine bevorzugte Ausrichtung einer auf dem Tisch 1 angeordneten Bildausgabevorrichtung (z.B. Monitor), beispielsweise die Höhe des Monitors sowie die Neigung der Bildfläche des Monitors bezüglich der Oberfläche der Tischplatte 18; eine bevorzugte Höhe einer vor dem Tisch 1 angeordneten Sitzeinrichtung (z.B. Schreibtischstuhl); eine bevorzugte Beleuchtungskonfiguration in der Umgebung des Tischs 1; Anmeldeinformationen des Benutzers zum Freigeben eines Computers oder einer Workstation; eine Telefonnummer (Durchwahl) des Benutzers; usw.

Alternativ zu der erwähnten kabellosen Funkschnittstelle (W-Lan und/oder Bluetooth) ist jeder beliebige Funkstandard in dem erfindungsgemäßen höhenverstellbaren Tisch 1 einsetzbar.

Fig. 2a zeigt eine mögliche Ausgestaltung des Bedienelements 15. In der hier dargestellten Ausführungsform weist das Bedienelement 15 einen Kippschalter K auf, der um eine horizontale Achse X nach oben bzw. nach unten gekippt werden kann. Der Kippschalter K ist dazu eingerichtet, mit einem Finger eines Benutzers bedient zu werden. Des Weiteren ist der Kippschalter K dazu eingerichtet, beim Loslassen des Kippschalters K in seine Ausgangslage (neutrale Lage) zurückzukehren, welche weder nach oben, noch nach unten gekippt ist. Der Kippschalter K weist des Weiteren einen Fingerabdrucksensor 151 auf, welcher dazu eingerichtet ist, den Fingerabdruck des Fingers zu erkennen, der den Kippschalter K berührt. Der Kippschalter K ist des Weiteren dazu eingerichtet, ein Signal an die Steuereinheit 14 zu übermitteln. Anhand dieses Signals erkennt die Steuereinheit 14, ob der Kippschalter K durch einen Finger berührt wird, welchen Fingerabdruck dieser Finger aufweist, und ob sich der Kippschalter K in neutraler Lage befindet, oder ob er nach oben oder unten gekippt ist.

Fig. 2b zeigt eine weitere mögliche Ausgestaltung des Bedienelements 15, wobei dieses Bedienelement 15 anstatt eines Kippschalters K zwei Erkennungsflächen (oder auch Knöpfe) E1, E2 aufweist. Jede dieser Erkennungsflächen E1, E2 weist einen Fingerabdrucksensor 151 auf. Jede dieser Erkennungsflächen E1, E2 ist des Weiteren dazu eingerichtet, ein Signal an die Steuereinheit 14 zu übermitteln. Anhand dieses Signals erkennt die Steuereinheit 14, ob eine Erkennungsfläche durch einen Finger berührt wird und welchen Fingerabdruck dieser Finger aufweist. Die Erkennungsflächen E1, E2 sind in dieser Ausführungsform horizontal nebeneinander angeordnet.

In einer weiteren, nicht gezeigten Ausführungsform sind die Erkennungsflächen E1, E2 drucksensitiv ausgeführt. In diesem Fall erkennt die Steuereinheit 14 des Weiteren, ob auf eine berührte Erkennungsfläche E1, E2 eine Kraft ausgeübt wird, d.h., ob der Finger die Erkennungsfläche E1, E2 nicht lediglich berührt, sondern diese auch drückt. Die restliche Konfiguration entspricht der Figurenbeschreibung zu Fig. 2b.

In einer weiteren, nicht gezeigten Ausführungsform sind die Erkennungsflächen E1, E2 vertikal übereinander angeordnet. Die restliche Konfiguration entspricht der Figurenbeschreibung zu Fig. 2b, oder alternativ der obigen Figurenbeschreibung zu der drucksensitiven Ausführung.

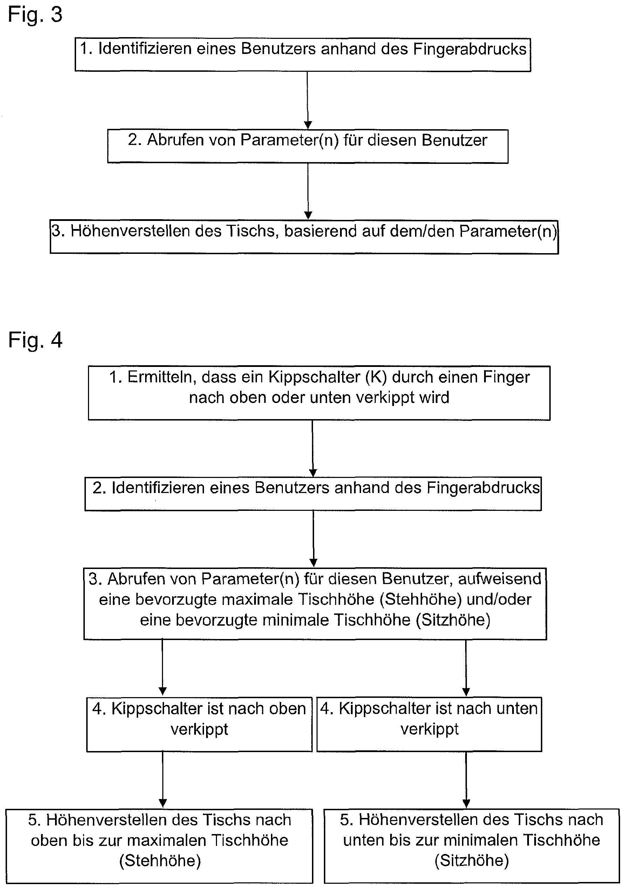

Fig. 3 zeigt ein grundlegendes Verfahren zum individualisierten Höhenverstellen eines Tischs, auf welchem die nachfolgend beschriebenen Verfahren basieren. In einem ersten Schritt wird ein Benutzer anhand seines Fingerabdrucks identifiziert. Dies geschieht beispielsweise durch das Erfassen des Fingerabdrucks mittels einem der oben beschriebenen Fingerabdrucksensoren 151 (des Kippschalters K bzw. der Erkennungsflächen E1, E2). Daraufhin wird der erfasste Fingerabdruck beispielsweise durch ein oben beschriebenes Steuergerät 14 mit (gespeicherten oder abgerufenen) Datensätzen verglichen (interne Kommunikation), um den/die zu genau diesem Benutzer gehörigen Parameter auszulesen. Der/die Parameter werden also in dem zweiten Schritt (siehe Fig. 3) für genau den Benutzer abgerufen, zu welchem der erfasste Fingerabdruck gehört. Daraufhin entscheidet das Steuergerät 14, welche Steueraktion basierend auf dem/den ausgelesenen Parameter(n) und basierend auf der konkreten Bedienung durch den Finger des Benutzers auszuführen ist. Beispielsweise besteht die auszuführende Steueraktion aus dem Höhenverstellen eines oben beschriebenen Tischs 1, wie im dritten Schritt (siehe Fig. 3) dargestellt ist.

Fig. 4 zeigt im Vergleich zu dem Verfahren aus Fig. 3 ein detaillierteres Verfahren, um eine mögliche Ausgestaltung der Erfindung zu erläutern. Es wird ein Verfahren beschrieben, das beispielsweise unter Verwendung eines Tischs 1 mit einem Bedienelement 15 anwendbar ist, bei dem ein Kippschalter K mit einem Fingerabdrucksensor 151 vorgesehen ist, siehe Fig. 2a.

In einem ersten Schritt wird durch das Steuergerät 14 ermittelt, dass der Kippschalter K durch einen Finger eines Benutzers berührt wird. Des Weiteren wird in diesem ersten Schritt ermittelt, dass der Kippschalter K durch den Finger nach oben oder unten gekippt wird. In einem zweiten Schritt wird daraufhin der Benutzer anhand seines Fingerabdrucks identifiziert, analog zu dem oben beschriebenen Vorgehen bezüglich Fig. 3.

Alternativ hierzu kann der Benutzer bereits in dem ersten Schritt identifiziert werden, noch bevor ein Verkippen des Schalters K erkannt wird. Bei dieser Alternative wird dann erst in dem zweiten Schritt ermittelt, dass der Kippschalter K durch den Finger nach oben oder unten gekippt wird.

In einem dritten Schritt werden daraufhin Parameter für den erkannten Benutzer abgerufen, beispielsweise durch das Steuergerät 14, wie unter Bezugnahme auf Fig. 3 erläutert wurde. In der vorliegenden Ausführungsform weisen die abgerufenen Parameter eine bevorzugte Tischhöhe zum Stehen und eine bevorzugte Tischhöhe zum Sitzen auf. In der vorliegenden Ausführungsform wird außerdem angenommen, dass die ursprüngliche Tischhöhe zwischen dieser bevorzugten Tischhöhe zum Stehen und dieser bevorzugte Tischhöhe zum Sitzen liegt.

Im darauffolgenden vierten Schritt trennen sich die Pfade des in Fig. 4 gezeigten Ablaufdiagramms. Falls sich der Kippschalter K in einem nach oben gekippten Zustand befindet, entspricht der weitere Ablauf des Verfahrens dem linken Pfad. Falls sich der Kippschalter K in einem nach unten gekippten Zustand befindet, entspricht der weitere Ablauf des Verfahrens dem rechten Pfad.

Im darauffolgenden fünften Schritt wird ein Höhenverstellen des Tischs 1 nach oben gestartet, in Richtung der bevorzugten Tischhöhe zum Stehen, falls der Ablauf dem linken Pfad entspricht. Andernfalls, falls der Ablauf dem rechten Pfad entspricht, wird ein Höhenverstellen des Tischs 1 nach unten gestartet, in Richtung der bevorzugten Tischhöhe zum Sitzen. Das Höhenverstellen wird so lange ausgeführt, bis die jeweilige bevorzugte Tischhöhe erreicht ist. Daraufhin wird das Höhenverstellen automatisch gestoppt. Das Höhenverstellen wird außerdem auch dann gestoppt, wenn der Kippschalter K durch den Benutzer losgelassen wird. Dies ist dadurch begründet, dass gesetzliche Vorgaben aus Sicherheitsaspekten ein automatisches Verstellen eines höhenverstellbaren Tischs zum jetzigen Zeitpunkt in vielen Ländern verbieten. Aus technischer Sicht allerdings ist dieses Problem lösbar, wie nachfolgend erläutert.

In einer nicht dargestellten Ausführungsform wird das Höhenverstellen des Tischs 1 nicht gestoppt, wenn der Kippschalter K durch den Benutzer losgelassen wird. In dieser Ausführungsform ist darüber hinaus ein Kollisionssensor vorgesehen, der eine Kollision der Tischplatte 18 beim Höhenverstellen erkennt. In dem dazugehörigen Verfahren wird hierbei während des Verstellvorgangs eine Kollision der Tischplatte 18 mit Gegenständen überwacht. Bei einer erkannten Kollision wird der Verstellvorgang unmittelbar abgebrochen.

Die obige Annahme, dass die ursprüngliche Tischhöhe zwischen der bevorzugten Tischhöhe zum Stehen und der bevorzugten Tischhöhe zum Sitzen liegt, wurde getroffen, um den grundlegenden Erfindungsgedanken vereinfacht zu erklären. Falls diese Annahme nicht zutrifft, sind Abweichungen im Verfahren vorgesehen. In dieser Ausführungsform wird bei einem Verkippen des Kippschalters K nach oben immer die bevorzugte Tischhöhe zum Stehen angefahren, selbst dann, wenn die ursprüngliche Tischhöhe darüber liegt. Hierbei ist der Kippschalter folglich nach oben gekippt, obwohl daraufhin ein Höhenverstellen des Tischs 1 nach unten ausgeführt wird. Analog dazu verhält sich der Zustand, in dem die ursprüngliche Tischhöhe unter der bevorzugten Tischhöhe zum Sitzen liegt.

In einer anderen, nicht gezeigten Ausführungsform wird bei einem ersten Verkippen des Kippschalters K nach oben immer ein Höhenverstellen des Tischs 1 nach oben ausgeführt (analog dazu bei einem ersten Verkippen des Kippschalters K nach unten, ein Höhenverstellen nach unten).

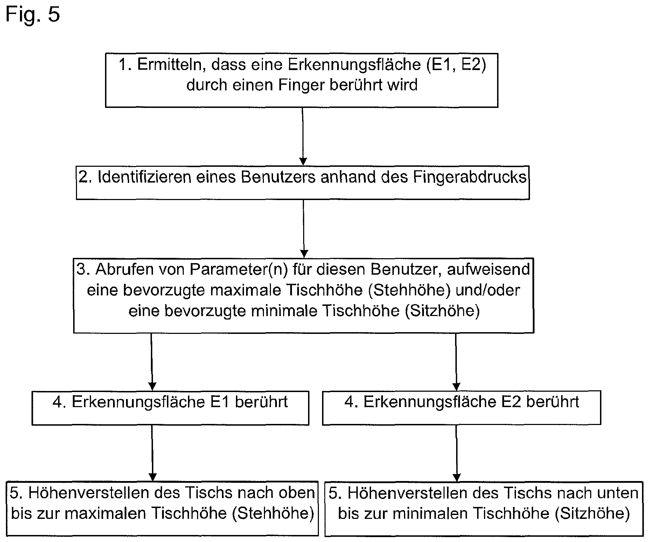

Fig. 5 zeigt im Vergleich zu dem Verfahren aus Fig. 4 ein Verfahren, das beispielsweise unter Verwendung eines Tischs 1 mit einem Bedienelement 15 anwendbar ist, bei dem zwei Erkennungsflächen E1, E2 mit jeweils einem Fingerabdrucksensor 151 vorgesehen sind, siehe Fig. 2b.

Das in Fig. 5 dargestellte Verfahren funktioniert analog zu dem in Fig. 4 dargestellten Verfahren, mit dem Unterschied, dass nicht ein Verkippen eines Kippschalters K erkannt wird, sondern anstatt dessen ein Betätigen von wenigstens einer der Erkennungsflächen E1, E2 durch wenigstens einen Finger eines Benutzers. Des Weiteren ist in der in Fig. 5 gezeigten Ausführungsform das Berühren der Erkennungsfläche E1 der Funktion "Höhenverstellung auf bevorzugte Tischhöhe zum Stehen", und die Erkennungsfläche E2 der Funktion "Höhenverstellung auf bevorzugte Tischhöhe zum Sitzen" zugeordnet.

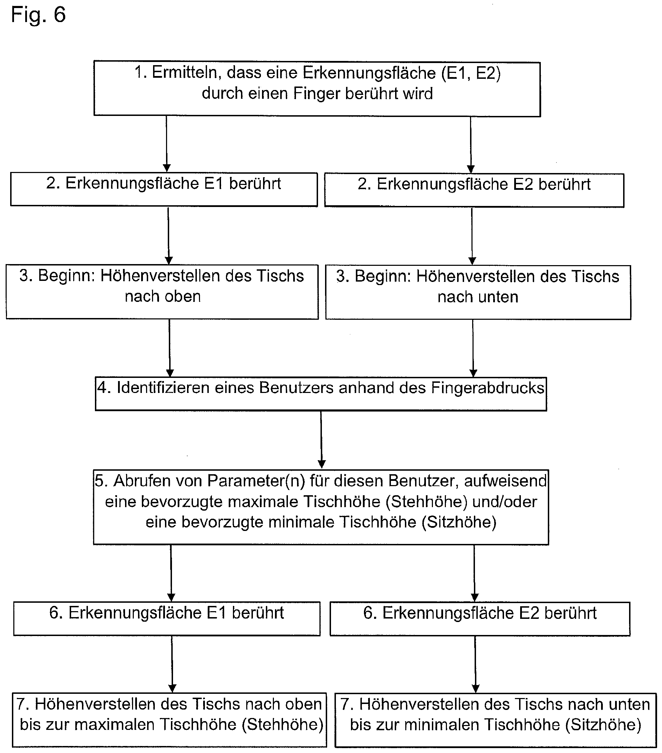

Fig. 6 zeigt eine Ausführungsform, bei welcher das Identifizieren des Benutzers anhand seines Fingerabdrucks nicht sofort in Schritt 1 oder 2 ausgeführt wird, sondern erst in einem späteren Schritt.

Bei dieser Ausführungsform wird im ersten Schritt ermittelt, dass eine der Erkennungsflächen E1, E2 durch einen Finger eines Benutzers berührt wird. Im darauffolgenden nächsten Schritt, dem zweiten Schritt, trennen sich bereits die Pfade des Verfahrens. In dieser Ausführungsform ist dem Betätigen der Erkennungsfläche E1 die Funktion "Höhenverstellung auf bevorzugte Tischhöhe zum Stehen" zugeordnet, und dem Betätigen der Erkennungsfläche E2 die Funktion "Höhenverstellung auf bevorzugte Tischhöhe zum Sitzen".

Diese Zuordnung kann in einer weiteren Ausführungsform umgekehrt sein, des Weiteren können den Erkennungsflächen E1, E2 auch andere Funktionen zugeordnet werden.

Wenn bei dieser Ausführungsform ermittelt wird, dass die Erkennungsfläche E1 berührt wird (Schritt 1 bzw. Schritt 2), wird bereits im dritten Schritt mit dem Höhenverstellen des Tischs nach oben begonnen. Zu diesem Zeitpunkt wurde noch nicht ermittelt, welcher Benutzer den Tisch aktuell bedient, und auf welcher Höhe die bevorzugten Positionen zum Sitzen/Stehen dieses Benutzers liegen. Erst im vierten Schritt, während des Höhenverstellens des Tischs, wird der Benutzer anhand seines Fingerabdrucks identifiziert. Daraufhin werden im fünften Schritt die Parameter für diesen Benutzer abgerufen, wie beispielsweise die bevorzugte Tischhöhe zum Sitzen, und die bevorzugte Tischhöhe zum Stehen. Falls der Tisch nun nach oben verstellt wird, also die Erkennungsfläche E1 berührt wird (siehe Schritt 6, bzw. auch bereits Schritt 2 und 3) wird das Höhenverstellen so lange ausgeführt, bis die bevorzugte Tischhöhe zum Stehen erreicht ist. Die Funktionsweise zum Höhenverstellen nach unten, durch Berühren der Erkennungsfläche E2, auf eine bevorzugte Tischhöhe zum Sitzen, funktioniert analog.

In dieser Ausführungsform beginnt das Höhenverstellen also bereits unmittelbar nach Betätigen einer der Erkennungsflächen E1, E2. Das Identifizieren des Benutzers und Auslesen der zu dem Benutzer gehörigen Parameter erfolgt erst danach. Vorzugsweise sind das Identifizieren des Benutzers sowie das Auslesen der Parameter in weniger als einer Sekunde nach Betätigen einer der Erkennungsflächen E1, E2 abgeschlossen. Diese Funktionsweise ist auch auf die Ausführungsform mit Kippschalter K zu adaptieren. Falls des Weiteren ein Identifizieren des Benutzers in Schritt 4 nicht möglich ist, wird das Höhenverstellen gemäß Schritt 3 so lange ausgeführt, bis entweder eine Endlage der Tischplatte 18 erreicht ist, oder bis die Erkennungsfläche E1, E2 nicht mehr berührt wird.

Wie bereits ausgeführt, beginnt in dieser Ausführungsform das Höhenverstellen bereits unmittelbar nach Betätigen einer der Erkennungsflächen E1, E2, noch bevor der Benutzer identifiziert wurde. Falls der Benutzer beispielsweise die Erkennungsfläche E1 berührt, um den Tisch auf eine bevorzugte Stehhöhe zu verstellen, beginnt ein Höhenverstellen der Tischplatte 18 nach oben, in Richtung der bevorzugten Stehhöhe, noch bevor der Benutzer identifiziert wurde. Falls hierbei allerdings zwischen Schritt 3 und Schritt 7 eine solche Zeitverzögerung auftritt, dass das Höhenverstellen der Tischplatte 18 zwar korrekterweise in Schritt 3 nach oben gestartet wurde, die Tischplatte 18 vor oder beim Ausführen des Schritts 7 allerdings bereits eine zu hohe Höhe erreicht hat, stoppt das Höhenverstellen der Tischplatte 18 gemäß einer vorteilhaften Weiterbildung des Verfahrens. Wenn der Benutzer daraufhin die Erkennungsfläche E1 weiterhin berührt, kehrt sich die Bewegungsrichtung der Tischplatte 18 um, und es beginnt ein Höhenverstellen der Tischplatte 18 nach unten, hin zur bevorzugten Stehhöhe.

In einer Weiteren, nicht dargestellten Ausführungsform ist ein beliebiges der oben beschriebenen Verfahren so ausgestaltet, dass der Tisch 1 durch Verkippen eines Kippschalters K nach oben bzw. durch Betätigen der Erkennungsfläche E1 generell nach oben verstellt wird, unabhängig von der Ausgangshöhe der Tischplatte 18, und durch Verkippen des Kippschalters K nach unten bzw. durch Betätigen der Erkennungsfläche E2 generell nach unten verstellt wird, unabhängig von der Ausgangshöhe der Tischplatte 18. Dabei stellen die für den Benutzer gespeicherten Parameter "bevorzugte Tischhöhe zum Sitzen" bzw. "bevorzugte Tischhöhe zum Stehen" lediglich Haltepunkte dar. Falls sich ein Tisch 1 beispielsweise in einer vollständig nach unten gefahrenen Position befindet, und ein Benutzer den Kippschalter K nach oben gekippt bzw. die Erkennungsfläche E1 berührt, wird ein Höhenverstellen des Tischs 1 bzw. der Tischplatte 18 nach oben ausgeführt. Je nach Ausgestaltung des Verfahrens (siehe oben) wurde bereits vor dem Beginn des Höhenverstellens oder erst danach der Benutzer identifiziert und die zu diesem gehörenden Parameter ermittelt. Wenn nun beim Höhenverstellen aus einer niedrigsten, maximal eingefahrenen Position die "bevorzugte Tischhöhe zum Sitzen" für den aktuellen Benutzer überfahren wird, stoppt der Tisch 1 automatisch an dieser Position (=Haltepunkt). Der Benutzer kann den Kippschalter K bzw. die Erkennungsfläche E1 nun loslassen. Der Tisch 1 bleibt daraufhin in seiner aktuellen Position stehen. Alternativ dazu kann der Benutzer den Kippschalter K aber auch nach oben gekippt halten (oder erneut nach oben kippen), bzw. weiter auf die Erkennungsfläche E1 drücken (oder diese erneut drücken). Daraufhin setzt sich der Tisch 1 nach einer kurzen Pause, beispielsweise nach 2 Sekunden, wieder in Bewegung, und setzt das Höhenverstellen nach oben fort, bis die "bevorzugte Tischhöhe zum Stehen" erreicht ist. Diese Höhe stellt hierbei wieder einen Haltepunkt dar. Analog dazu kann der Tische 1 daraufhin weiter nach oben verstellt werden, bis zu einer höchsten, maximal ausgefahrenen Position. Ein Höhenverstellen nach unten funktioniert analog hierzu.

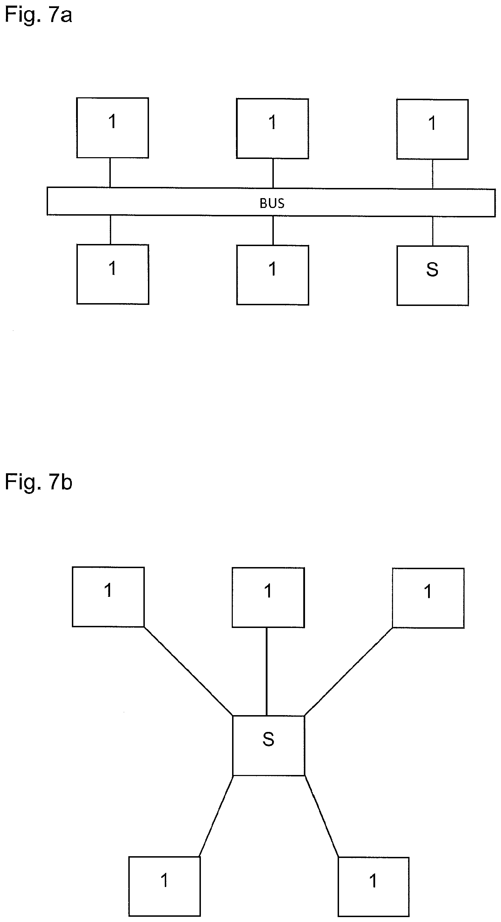

Fig. 7a zeigt eine Ausführungsform für ein System, bei welchem mehrere höhenverstellbare Schreibtische 1 vorgesehen sind, wobei jeder Schreibtisch 1 über ein Steuergerät 14 verfügt. Die Schreibtische 1 sind über ein BUS-System mit einem Server S verbunden, wobei jedes Steuergerät 14 dazu eingerichtet ist, über das BUS-System mit dem Server S zu kommunizieren. Beispielsweise können durch ein Steuergerät 14 Datensätze von dem Server S abgerufen werden. Jeder dieser Datensätze weist wenigstens einen gespeicherten Fingerabdruck eines jeweiligen Benutzers und weitere Parameter für diesen Benutzer auf. Diese Parameter weisen wenigsten einen der nachfolgenden Werte auf: Bevorzugte Tischplattenhöhe zum Stehen; Bevorzugte Tischplattenhöhe zum Sitzen; Bevorzugte Höhe eines Monitors zum Stehen; Bevorzugte Höhe eines Monitors zum Sitzen; Bevorzugte Neigung eines Monitors zum Stehen; Bevorzugte Neigung eines Monitors zum Sitzen; Bevorzugte Höhe eines Stuhls; Bevorzugte Beleuchtungskonfiguration; Zugangsdaten zum Anmelden an einem Computer oder an einer Workstation; Telefonnummer bzw. Durchwahl.

Fig. 7b zeigt einen alternativen Aufbau des Systems aus Fig. 7a. Bei diesem Aufbau ist die Verbindung zwischen den Schreibtischen 1 und dem Server S nicht über ein BUS-System realisiert, sondern über eine Sternschaltung. Die Funktionsweise ist sonst mit dem Aufbau aus Fig. 7a identisch.

In einer weiteren, nicht dargestellten Ausführungsform weist der Tisch 1 alternativ oder zusätzlich zu dem Bedienelement 15 eine Schnittstelle zu einem mobilen Bedienelement auf. Dieses mobile Bedienelement weist grundlegend die gleiche Funktionalität auf wie das Bedienelement 15, wobei das mobile Bedienelement aber einem festgelegten Benutzer zugeordnet ist. Folglich kann der Benutzer dadurch identifiziert werden, dass die Identität des mobilen Bedienelements ermittelt wird. Des Weiteren können in dem mobilen Bedienelement Parameter gespeichert sein, welche dem Benutzer zugeordnet sind, wie beispielsweise eine bevorzugte Tischhöhe zum Stehen. Des Weiteren könnte das mobile Bedienelement als Smartphone, Tablet etc. mit einer entsprechenden App ausgebildet sein. Vorzugsweise wird die Schnittstelle zwischen Tisch 1 und dem mobilen Bedienelement, bzw. zwischen Steuergerät 14 und dem mobilen Bedienelement, mittels Nahbereichserkennung (RFID) realisiert.

## Patentansprüche

1. Höhenverstellbarer Tisch (1), aufweisend
wenigstens eine elektrisch höhenverstellbare Tischsäule (12, 13), und
ein Steuergerät (14),
**dadurch gekennzeichnet, dass**
der höhenverstellbare Tisch (1) ein Bedienelement (15) mit wenigstens einem Fingerabdrucksensor (151) aufweist, wobei
der Fingerabdrucksensor (151) kombiniert mit einem Kippschalter (K) oder als Kippschalter (K) vorgesehen ist, der zum Kippen um eine Achse (X) nach oben und unten, bedienbar durch einen Finger, eingerichtet ist.

2. Höhenverstellbarer Tisch (1), aufweisend
wenigstens eine elektrisch höhenverstellbare Tischsäule (12, 13), und
ein Steuergerät (14),
**dadurch gekennzeichnet, dass**
der höhenverstellbare Tisch (1) ein Bedienelement (15) mit wenigstens zwei Erkennungsflächen (E1, E2) mit jeweils einem Fingerabdrucksensor (151) aufweist, wobei jede Erkennungsfläche (E1, E2) zum Bedienen einer Verstellfunktion des Tischs (1) vorgesehen ist.

3. Höhenverstellbarer Tisch (1) gemäß einem der vorhergehenden Ansprüche, wobei das Bedienelement (15) baulich in das Steuergerät (14) integriert ist.

4. Verfahren zum Höhenverstellen eines Tischs (1), gemäß Anspruch 1 oder 2, aufweisend die Schritte:
- Identifizieren eines Benutzers anhand eines Fingerabdrucks mittels eines Fingerabdrucksensors (151),
- Abrufen von wenigstens einem Parameter für diesen Benutzer,
- Höhenverstellen des Tischs (1), basierend auf dem Parameter und der Identität des Benutzers.

5. Verfahren gemäß des vorhergehenden Anspruchs zum Höhenverstellen eines Tischs (1) gemäß Anspruch 1, zusätzlich aufweisend die Schritte:
- Ermitteln, dass ein Kippschalter (K) durch einen Finger eines Benutzers nach oben oder unten gekippt wird,
- Identifizieren des Benutzers anhand des Finderabdrucks des Fingers, mittels eines Fingerabdrucksensors (151) des Kippschalters (K),
- Abrufen von Parametern für den ermittelten Benutzer, aufweisend eine bevorzugte maximale Tischhöhe (Stehhöhe) und/oder eine bevorzugte minimale Tischhöhe (Sitzhöhe),
- Verstellen des Tischs (1) in Richtung der bevorzugten maximalen Höhe, falls der Kippschalter (K) nach oben gekippt wird, bzw. Verstellen des Tischs (1) in Richtung der bevorzugten minimalen Höhe, falls der Kippschalter (K) nach unten gekippt wird.

6. Verfahren gemäß Anspruch 4 zum Höhenverstellen eines Tischs (1) gemäß Anspruch 2, zusätzlich aufweisend die Schritte:
- Ermitteln, dass eine Erkennungsfläche (E1, E2) durch einen Finger berührt wird, und Ermitteln durch einen Fingerabdrucksensor (151), welche Erkennungsfläche (E1, E2) dies ist,
- Identifizieren des Benutzers anhand des Finderabdrucks des Fingers, mittels des Fingerabdrucksensors (151),
- Abrufen von Parameters für den ermittelten Benutzer, beispielsweise aufweisend eine bevorzugte maximale Tischhöhe (Stehhöhe) und/oder eine minimale Tischhöhe (Sitzhöhe),
- Verstellen des Tischs (1) basierend auf einer Verstellfunktion, welche der berührten Erkennungsfläche (E1, E2) zugeordnet ist, und basierend auf den abgerufenen Parametern, beispielsweise Verstellen des Tischs (1) in Richtung der bevorzugten maximalen Höhe oder in Richtung der bevorzugten minimalen Höhe.

7. Verfahren gemäß einem der Ansprüche 5 oder 6, wobei
das Verstellen des Tischs (1) basierend auf der Verkipprichtung des Kippschalters (K) bzw. basierend auf der Identität der berührten Erkennungsfläche (E1, E2) bereits gestartet wird, noch bevor der Benutzer anhand seines Fingerabdrucks identifiziert wurde, bzw. noch bevor die Parameter für den erkannten Benutzer abgerufen wurden.

8. Verfahren gemäß des vorhergehenden Anspruchs, wobei
das Verstellen des Tischs (1) unmittelbar nach Verkippen des Kippschalters (K) bzw. unmittelbar nach Berühren der Erkennungsfläche (E1, E2) gestartet wird.

9. Verfahren zum Höhenverstellen eines Tischs gemäß Anspruch 1 oder 2, aufweisend die Schritte:
- Identifizieren eines Fingers eines Benutzers anhand des Fingerabdrucks mittels eines Fingerabdrucksensors (151),
- Abrufen einer Funktion für genau diesen Finger dieses Benutzers,
- Ausführen der Funktion.

10. Verfahren gemäß einem der Ansprüche 4 bis 9, wobei anhand eines ermittelten Benutzers bzw. anhand eines ermittelten Fingers eines Benutzers, basierend auf abgerufenen Parametern und einem aktuellen Zustand, weitere Funktionen ausgeführt werden, die beispielsweise aufweisen:
- Höhenverstellen einer Tischplatte (18) nach oben,
- Höhenverstellen einer Tischplatte (18) nach unten,
- Höhenverstellen eines Monitors nach oben,
- Höhenverstellen eines Monitors nach unten,
- Kippen eines Monitors nach vorne,
- Kippen eines Monitors nach hinten,
- Höhenverstellen eines Stuhls nach oben,
- Höhenverstellen eines Stuhls nach unten,
- Licht an,
- Licht aus,
- Anmelden und/oder Freigeben eines Computers oder einer Workstation,
- Sperren eines Computers oder einer Workstation, und
- Umschalten eines Telefons auf die Durchwahl des ermittelten Benutzers.

11. Küche mit einer Arbeitsplatte, wobei die Arbeitsplatte als höhenverstellbarer Tisch (1) gemäß einem der Ansprüche 1 bis 3 ausgeführt ist.

12. System aus wenigstens zwei höhenverstellbaren Tischen (1) gemäß einem der Ansprüche 1 bis 3 und einem Server (S), wobei
der Server (S) dazu eingerichtet ist, Daten zu verwalten, welche von den Steuergeräten (14) der höhenverstellbaren Tische (1) abrufbar sind.

13. System gemäß des vorhergehenden Anspruchs, wobei
die Daten aus wenigstens zwei Datensätzen bestehen, wobei jeder Datensatz einem Benutzer zugeordnet ist, wobei jeder Datensatz wenigstens einen gespeicherten Fingerabdruck des jeweiligen Benutzers und weitere Parameter für den Benutzer aufweist.

14. System gemäß des vorhergehenden Anspruchs, wobei die Parameter wenigsten einen der nachfolgenden Werte aufweisen:
- Bevorzugte Tischplattenhöhe zum Stehen,
- Bevorzugte Tischplattenhöhe zum Sitzen,
- Bevorzugte Höhe eines Monitors zum Stehen,
- Bevorzugte Höhe eines Monitors zum Sitzen,
- Bevorzugte Neigung eines Monitors zum Stehen,
- Bevorzugte Neigung eines Monitors zum Sitzen,
- Bevorzugte Höhe eines Stuhls,
- Bevorzugte Beleuchtungskonfiguration,
- Zugangsdaten zum Anmelden an einem Computer oder an einer Workstation, und
- Telefonnummer bzw. Durchwahl.

## Claims

1. Height adjustable table (1), comprising
at least one electrically height adjustable table column (12, 13), and
a control device (14),
**characterized in that**
the height adjustable table (1) comprises an operating element (15) having at least one fingerprint sensor (151), wherein
the fingerprint sensor (151) is provided in combination with a flip switch (K) or as flip switch (K), which is set up to tilt around an axis (X) upward or downward and which can be operated by a finger.

2. Height adjustable table (1), comprising
at least one electrically height adjustable table column (12, 13), and
a control device (14),
**characterized in that**
the height adjustable table (1) comprises an operating element (15) with at least two detection areas (E1, E2), each having a fingerprint sensor (151), wherein each detection area (E1, E2) is provided to operate an adjustment function of the table (1).

3. Height adjustable table (1) according to anyone of the preceding claims, wherein the operating element (15) is structurally integrated into the control device (14).

4. Method for height adjustment of a table (1) according to claim 1 or 2, comprising the steps:
- identification of a user using a fingerprint by means of a fingerprint sensor (151),
- retrieving at least one parameter for this user,
- height adjustment of the table (1) based on the parameter and the identity of the user.

5. Method according to the preceding claim for height adjustment of a table (1) according to claim 1, furthermore comprising the steps:
- determining that a flip switch (K) is tilted upwards or downwards by a finger of a user,
- identifying the user using the fingerprint of the finger by means of a fingerprint sensor (151) of the flip switch (K),
- retrieving parameters for the identified user, comprising a preferred maximum table height (standing height) and/or a preferred minimum table height (sitting height),
- adjusting the table (1) in direction of the preferred maximum height if the flip switch (K) is tilted upwards, or adjusting the table (1) in direction of the preferred minimum height if the flip switch (K) is tilted downwards.

6. Method according to claim 4 for height adjustment of a table (1) according to claim 2, furthermore comprising the steps:
- determining that a detection area (E1, E2) is touched by a finger, and determining which detection area (E1, E2) that is by means of a fingerprint sensor (151),
- identifying the user using the fingerprint of the finger by means of the fingerprint sensor (151),
- retrieving parameters for the identified user, comprising, for example, a preferred maximum table height (standing height) and/or a minimum table height (sitting height),
- adjusting the table (1) based on an adjustment function which is assigned to the touched detection area (E1, E2) and based on the retrieved parameters, e.g., adjustment of the table (1) in direction of the preferred maximum height or in direction of the preferred minimum height.

7. Method according to anyone of claims 5 or 6, wherein
the adjustment of the table (1) based on the tilting direction of the flip switch (K) or based on the identity of the touched detection area (E1, E2) is already started, even before the user has been identified by his fingerprint, or before the parameters for the identified user have been retrieved.

8. Method according to the preceding claim, wherein
the adjustment of the table (1) is started immediately after tilting the flip switch (K) or immediately after touching the detection area (E1, E2).

9. Method for height adjustment of a table according to claim 1 or 2, comprising the steps:
- identifying a finger of a user using the fingerprint by means of a fingerprint sensor (151),
- retrieving a function for exactly this finger of this user,
- executing the function.

10. Method according to anyone of claims 4 to 9, wherein,
by means of the identified user or by means of an identified finger of a user, based on retrieved parameters and a current status, further functions are performed, which comprise for example:
- height adjustment of a table top (18) upward,
- height adjustment of a table top (18) downward,
- height adjustment of a monitor upward,
- height adjustment of a monitor downward,
- tilting of a monitor forward,
- tilting of a monitor backward,
- height adjustment of a chair upward,
- height adjustment of a chair downward,
- light on,
- light off,
- login and/or releasing a computer or a workstation
- locking a computer or a workstation, and
- switching a telephone to the extension of the ascertained user.

11. Kitchen having a worktop, wherein
the worktop is designed as a height adjustable table (1) according to anyone of the claims 1 to 3.

12. System of at least two height adjustable tables (1) according to anyone of claims 1 to 3 and a server (S), wherein
the server (S) is set up to manage data retrievable from the control devices (14) of the height adjustable tables (1).

13. System according to the preceding claim, wherein
the data consist of at least two sets of data, wherein each data set is assigned to a user, wherein each data set comprises at least one stored fingerprint of the respective user as well as further parameters for the user.

14. System according to the preceding claim, wherein
the parameters comprise at least one of the following values:
- preferred table top height for standing,
- preferred table top height for sitting,
- preferred height of a monitor for standing,
- preferred height of a monitor for sitting,
- preferred tilting of a monitor for standing,
- preferred tilting of a monitor for sitting,
- preferred height of a chair,
- preferred lighting configuration,
- login data for logging in to a computer or a workstation, and
- telephone number or extension.

## Revendications

1. Table (1) ajustable en hauteur, présentant
au moins une colonne de table (12, 13) ajustable en hauteur électriquement, et
un appareil de commande (14),
**caractérisée en ce que**
la table (1) ajustable en hauteur présente un élément de commande (15) avec au moins un capteur d'empreinte digitale (151), dans laquelle
le capteur d'empreinte digitale (151) est prévu de manière combinée avec un interrupteur à bascule (K) ou en tant qu'interrupteur à bascule (K), qui est mis au point pour basculer autour d'un axe (X) vers le haut et vers le bas, tout en pouvant être commandé par un doigt.

2. Table (1) ajustable en hauteur, présentant
au moins une colonne de table (12, 13) ajustable en hauteur électriquement, et
un appareil de commande (14),
**caractérisée en ce que**
la table (1) ajustable en hauteur présente un élément de commande (15) avec au moins deux surfaces d'identification (E1, E2) avec respectivement un capteur d'empreinte digitale (151), dans laquelle chaque surface d'identification (E1, E2) est prévue pour commander une fonction d'ajustement de la table (1).

3. Table (1) ajustable en hauteur selon l'une quelconque des revendications précédentes, dans laquelle l'élément de commande (15) est intégré de manière structurelle dans l'appareil de commande (14).

4. Procédé pour ajuster en hauteur une table (1) selon la revendication 1 ou 2, présentant les étapes :
- d'identification d'un utilisateur à l'aide d'une empreinte digitale au moyen d'un capteur d'empreinte digitale (151),
- d'appel d'au moins un paramètre pour ledit utilisateur,
- d'ajustement en hauteur de la table (1) sur la base du paramètre et de l'identité de l'utilisateur.

5. Procédé selon la revendication précédente pour ajuster en hauteur une table (1) selon la revendication 1, présentant en supplément les étapes :
- de détermination qu'un interrupteur à bascule (K) est basculé vers le haut ou le bas par un doigt d'un utilisateur,
- d'identification de l'utilisateur à l'aide de l'empreinte digitale du doigt, au moyen d'un capteur d'empreinte digitale (151) de l'interrupteur à bascule (K),
- d'appel de paramètres pour l'utilisateur déterminé, présentant une hauteur de table maximale préférée (hauteur debout) et/ou une hauteur de table minimale préférée (hauteur d'assise),
- d'ajustement de la table (1) en direction de la hauteur maximale préférée si l'interrupteur à bascule (K) est basculé vers le haut ou d'ajustement de la table (1) en direction de la hauteur minimale préférée si l'interrupteur à bascule (K) est basculé vers le bas.

6. Procédé selon la revendication 4 pour ajuster en hauteur une table (1) selon la revendication 2, présentant en supplément les étapes :
- de détermination qu'une surface d'identification (E1, E2) est touchée par un doigt, et de détermination par un capteur d'empreinte digitale (151) de la surface d'identification (E1, E2) dont il s'agit,
- d'identification de l'utilisateur à l'aide de l'empreinte digitale du doigt au moyen du capteur d'empreinte digitale (151),
- d'appel de paramètres pour l'utilisateur déterminé, par exemple présentant une hauteur de table maximale préférée (hauteur debout) et/ou une hauteur de table minimale (hauteur d'assise),
- d'ajustement de la table (1) sur la base d'une fonction d'ajustement, laquelle est associée à la surface d'identification (E1, E2) touchée, et sur la base des paramètres appelés, par exemple d'ajustement de la table (1) en direction de la hauteur maximale préférée ou en direction de la hauteur minimale préférée.

7. Procédé selon l'une quelconque des revendications 5 ou 6, dans lequel l'ajustement de la table (1) commence déjà sur la base de la direction de basculement de l'interrupteur à bascule (K) ou sur la base de l'identité de la surface d'identification (E1, E2) touchée avant que l'utilisateur n'ait été identifié à l'aide de son empreinte digitale ou avant que les paramètres n'aient été appelés pour l'utilisateur identifié.

8. Procédé selon la revendication précédente, dans lequel
l'ajustement de la table (1) démarre directement après le basculement de l'interrupteur à bascule (K) ou directement après le contact avec la surface d'identification (E1, E2).

9. Procédé pour ajuster en hauteur une table selon la revendication 1 ou 2, présentant les étapes :
- d'identification d'un doigt d'un utilisateur à l'aide de l'empreinte digitale au moyen d'un capteur d'empreinte digitale (151),
- d'appel d'une fonction pour précisément ledit doigt dudit utilisateur,
- d'exécution de la fonction.

10. Procédé selon l'une quelconque des revendications 4 à 9, dans lequel sont exécutées, à l'aide d'un utilisateur déterminé ou à l'aide d'un doigt déterminé d'un utilisateur, sur la base de paramètres appelés et d'un état actuel, d'autres fonctions, qui présentent par exemple :
- l'ajustement en hauteur d'un plateau de table (18) vers le haut,
- l'ajustement en hauteur d'un plateau de table (18) vers le bas,
- l'ajustement en hauteur d'un moniteur vers le haut,
- l'ajustement en hauteur d'un moniteur vers le bas,
- le basculement d'un moniteur vers l'avant,
- le basculement d'un moniteur vers l'arrière,
- l'ajustement en hauteur d'une chaise vers le haut,
- l'ajustement en hauteur d'une chaise vers le bas,
- de la lumière on,
- de la lumière off,
- la connexion et/ou la déconnexion d'un ordinateur ou d'un poste de travail,
- l'arrêt d'un ordinateur ou d'un poste de travail, et
- la commutation d'un téléphone sur l'appel direct de l'utilisateur déterminé.

11. Cuisine avec un plan de travail, dans laquelle le plan de travail est réalisé en tant qu'une table (1) ajustable en hauteur selon l'une quelconque des revendications 1 à 3.

12. Système composé d'au moins deux tables (1) ajustables en hauteur selon l'une quelconque des revendications 1 à 3 et d'un serveur (S), dans lequel
le serveur (S) est mis au point pour gérer des données, lesquelles peuvent être appelées par les appareils de commande (14) des tables (1) ajustables en hauteur.

13. Système selon la revendication précédente, dans lequel
les données sont constituées d'au moins deux jeux de données, dans lequel chaque jeu de données est associé à un utilisateur, dans lequel chaque jeu de données présente au moins une empreinte digitale mémorisée de l'utilisateur respectif et d'autres paramètres pour l'utilisateur.

14. Système selon la revendication précédente, dans lequel les paramètres présentent au moins une des valeurs suivantes :
- hauteur de plateau de table préférée pour être debout,
- hauteur de plateau de table préférée pour être assis,
- hauteur préférée d'un moniteur pour être debout,
- hauteur préférée d'un moniteur pour être assis,
- inclinaison préférée d'un moniteur pour être debout,
- inclinaison préférée d'un moniteur pour être assis,
- hauteur préférée d'une chaise,
- configuration d'éclairage préférée,
- données d'accès pour la connexion à un ordinateur ou à un poste de travail, et
- numéro de téléphone ou ligne directe.
